# EUROPÄISCHE PATENTSCHRIFT

(11) **EP 2 446 860 B1**
(45) Veröffentlichungstag und Bekanntmachung des Hinweises auf die Patenterteilung: **02.12.2015**
(21) Anmeldenummer: 10013959.1
(22) Anmeldetag: 26.10.2010
(51) Int. Cl.: A61F 2/44, A61F 2/46

(54) **Bandscheibenprothese**
Spinal disc prosthesis
Prothèse discale

(43) Veröffentlichungstag der Anmeldung: 02.05.2012
(73) Patentinhaber: Röbling, Christian, 79104 Freiburg (DE); Rieger, Claudia, 06108 Halle/Saale (DE); Hedayat, Farman, 50931 Köln (DE)
(72) Erfinder: Röbling, Christian, 79104 Freiburg (DE); Rieger, Claudia, 06108 Halle/Saale (DE); Hedayat, Farman, 50931 Köln (DE)
(74) Vertreter: Westphal, Mussgnug & Partner Patentanwälte mbB

(56) Entgegenhaltungen:
- EP-A1- 0 965 313
- WO-A1-00/25706
- WO-A1-00/40179
- WO-A1-2010/092613
- WO-A2-2004/037067
- FR-A1- 2 771 282
- US-A1- 2004 249 461
- US-A1- 2005 021 144
- US-A1- 2006 004 448
- US-A1- 2008 154 377
- US-A1- 2009 048 676
- US-A1- 2010 016 973
- US-B1- 6 368 351
- US-B1- 6 454 807

## Beschreibung

Die Erfindung betrifft eine Bandscheibenprothese.

Bekannt sind Bandscheibenprothesen zum Einsetzen in den Zwischenraum zwischen zwei benachbarten Wirbeln, welche eine defekte Bandscheibe ersetzen.

Verschiedene Bandscheibenprothesen werden beispielsweise in US 6 454 807 B1, US 2004/249461, US 2010/016973 A1, WO 00/25706 A1, US 2009/048676 A1, WO 2004/037067 A2, US 2005/021144 A1, US 6 368 351 B1, WO 2010/092613 A1, US 2006/004448 A1, EP 0 965 313 A1, WO 00/40179 A1, US 2008/154377 A1, FR 2 771 282 A1 gezeigt. WO2010/092613 beschreibt eine Bandscheibenprothese entsprechend der Präambel des Anspruchs 1.

Die Aufgabe der Erfindung besteht darin, eine Bandscheibenprothese bereitzustellen, welche einfach aufgebaut und flexibel einsetzbar ist.

Die Aufgabe wird erfindungsgemäß gelöst durch eine Bandscheibenprothese zum Einsetzen zwischen zwei benachbarten Wirbelkörpern mit den Merkmalen des Patentanspruchs 1.

Vorteilhafte Ausgestaltungen und Weiterbildungen der Erfindung sind in den abhängigen Ansprüchen angegeben.

Die erfindungsgemäße Bandscheibenprothese zum Einsetzen zwischen zwei benachbarten Wirbelkörpern zeichnet sich dadurch aus, dass die Bandscheibenprothese U-förmig ausgebildet ist mit ersten Schenkei und einem zweiten Schenkel, wobei die beiden Schenkel relativ zueinander verschwenkbar sind. Unter einer U-förmigen Form ist dabei insbesondere jede Geometrie zu verstehen, welcher zwei Elemente mit einer Längserstreckung aufweist, die an einem Ende oder in der Nähe eines Endes entweder direkt oder mittels eines Verbindungselements miteinander verbunden sind und deren anderes Ende als freies Ende ausgebildet ist. Unter anderem wird unter einer U-förmigen Geometrie beispielsweise auch ein geschlitzter Ring oder eine C-förmige Geometrie verstanden. Insbesondere können die beiden Schenkel zusätzliche Geometrien, insbesondere in Form von Vorsprüngen oder Ausnehmungen, aufweisen, die insbesondere an die anatomischen Gegebenheiten angepasst sind.

Die erfindungsgemäße Bandscheibenprothese weist den Vorteil auf, dass zum Einsetzen der Bandscheibenprothese die beiden Schenkel der U-förmigen Bandscheibenprothese möglichst eng aneinander liegen, sodass die Bandscheibenprothese durch einen kleinen Schnitt in der Haut eingeführt werden kann und anschließend im Zwischenwirbelraum die beiden Schenkel gegeneinander verschwenkt werden können, insbesondere in der Ebene der U-förmigen Bandscheibenprothese, welche insbesondere im implantierten Zustand quer zur Längsrichtung der Wirbelsäule verläuft, um durch das Aufschwenken die beiden benachbarten Wirbelkörper auf breiterer Fläche gegeneinander abzustützen. Insbesondere ermöglicht die U-förmige Ausgestaltung der Bandscheibenprothese das Einsetzen der Bandscheibenprothese, nachdem zwei benachbarte Wirbelkörper mittels einer Knochenschraube gegeneinander stabilisiert wurden, wobei die Knochenschraube den Zwischenwirbelraum quer durchsetzt. Die Bandscheibenprothese kann auch nach Einsetzen der Knochenschraube eingeführt werden derart, dass die beiden Schenkel der U-förmigen Bandscheibenprothese seitlich an der Knochenschraube vorbeigeführt werden und somit die beiden benachbarten Wirbel zuverlässig gegeneinander abstützt.

Gemäß einer bevorzugten Ausführungsform der Erfindung ist die Bandscheibeprothese aus einem elastischen Material gefertigt, welches insbesondere ein Verschwenken der beiden Schenkel relativ zueinander ermöglicht.

Gemäß einer vorteilhaften Ausführungsform der Erfindung sind die beiden Schenkel jeweils über ein Scharnier schwenkbar angeordnet oder jeweils mittels eines Bolzens schwenkbar gelagert angeordnet, um ein definiertes, stufenloses Verschwenken der beiden Schenkel relativ zueinander zu ermöglichen.

Vorzugsweise weisen die beiden Schenkel einen Zahnradabschnitt auf, in welcher eine Aufspreizvorrichtung, insbesondere mittels eines Zahnradsabschnitts, zum Schwenken eingreifen kann. Auf diese Art und Weise wird ein definierter Aufspreizvorgang ermöglicht.

Gemäß einer bevorzugten Ausführungsform der Erfindung sind die beiden Schenkel mittels eines Federelements, insbesondere eines Federelements aus einer Memorylegierung, verschwenkbar. Besonders bevorzugt ändert die Memorylegierung bei Erreichen der Körpertemperatur ihre Form, sodass beispielsweise die Bandscheibenprothese bei Raumtemperatur in den Zwischenwirbelraum gesetzt werden kann, wobei das Federelement derart angeordnet ist, dass die beiden Schenkel nahezu oder direkt aneinander liegen, und nach Einsetzen der Bandscheibenprothese, sobald die Bandscheibenprothese und das Federelement die Körpertemperatur erreicht haben, die beiden Schenkel mittels des Federelements gegeneinander verschwenkt werden, sobald die Memoreglegierung ihre Form ändert.

Vorteilhafterweise ist an einem der beiden Schenkel ein Hebelelement schwenkbar gelagert angeordnet, welches bei Drehung um eine Drehachse an einer an dem anderen der beiden Schenkel angeordneten Zwangskurve angreift, um die beiden Schenkel relativ zueinander verschwenken. Auf diese Art und Weise wird ein stufenloses Verschwenken der beiden Schenkel relativ zueinander auf einfache Art und Weise realisiert.

Gemäß einer bevorzugten Ausführungsform der Erfindung sind die freien Enden der Schenkel aufeinander zugebogen, sodass auch bei aufgespreizter Bandscheibenprothese eine zuverlässige Abstützung der beiden benachbarten Wirbelkörper sowohl in sagittaler Richtung als auch in seitlicher Richtung gewährleistet ist.

Vorzugsweise sind an der Bandscheibenprothese wenigstens zwei vorzugsweise drei, Röntgenkontrastmarker angeordnet, um das Einsetzen der Bandscheibenprothese visuell verfolgen zu können.

Vorzugsweise weisen die beiden Schenkel jeweils ein freies Ende auf, welches mit einem Röntgenkontrastmittel, beispielsweise mit Tantal beschichtet ist, um das Einsetzen der Bandscheibenprothese, welches mit den freien Enden der Schenkel voran erfolgt, zuverlässig visuell verfolgen zu können.

Gemäß einer bevorzugten Ausführungsform der Erfindung weist einer der beiden Schenkel in der Ebene der U-förmigen Bandscheibenprothese eine größere Breite auf als der andere der beiden Schenkel, um insbesondere auch bei einer asymmetrisch durch den Zwischenraum zwischen den beiden benachbarten Wirbel verlaufenden Knochenschraube eine zuverlässige Abstützung der beiden benachbarten Wirbel gegeneinander gewährleisten zu können.

Vorzugsweise weist die Bandscheibenprothese wenigstens eine Ausnehmung auf, in welcher ein Einsetzinstrument eingreifen kann, um die Bandscheibenprothese während des Einsetzvorgangs definiert halten zu können.

Gemäß einer besonders bevorzugten Ausführungsform der Erfindung ist das Einsetzinstrument gleichzeitig geeignet, nach Einsetzen der Bandscheibenprothese die beiden Schenkel der Bandscheibenprothese aufzuspreizen.

Die Erfindung wird anhand der nachfolgenden Figuren ausführlich erläutert.

Es zeigt:
- Fig. 1: eine schematische Darstellung eines ersten Ausführungsbeispiels einer Bandscheibenprothese,
- Fig. 2: eine schematische Ansicht eines zweiten Ausführungsbeispiels einer Bandscheibenprothese,
- Fig. 3: eine schematische Ansicht eines dritten Ausführungsbeispiels einer Bandscheibenprothese,
- Fig. 4: eine schematische Ansicht eines vierten Ausführungsbeispiels einer Bandscheibenprothese,
- Fig. 5: eine schematische Ansicht eines fünften Ausführungsbeispiels einer erfindungsgemäßen Bandscheibenprothese,
- Fig. 6: eine Seitenansicht der Bandscheibenprothese gemäß Figur 5,
- Fig. 7: die Bandscheibenprothese gemäß Figur 5 in aufgeschwenktem Zustand,
- Fig. 8: eine schematische perspektivische Darstellung eines sechsten Ausführungsbeispiels einer Bandscheibenprothese,
- Fig. 9: eine Draufsicht auf die Bandscheibenprothese gemäß Figur 8 in zusammengeklapptem Zustand,
- Fig. 10: die Bandscheibenprothese gemäß Figur 8 in aufgeschwenktem Zustand,
- Fig. 11: eine perspektivische Darstellung des Federelements der Bandscheibenprothese gemäß Figur 9,
- Fig. 12: eine perspektivische Darstellung der Bandscheibenprothese gemäß Figur 8 in aufgespreiztem Zustand,
- Fig. 13: eine Draufsicht auf die zerlegte Bandscheibenprothese gemäß Figur 8,
- Fig. 14: die Bandscheibenprothese gemäß Figur 8 in zusammengeklapptem Zustand, welche in einen Halter eingesetzt ist,
- Fig. 15: die Bandscheibenprothese gemäß Figur 8 in aufgespreiztem Zustand, welche in einen Halter eingesetzt ist,
- Fig. 16: die Bandscheibenprothese gemäß Figur 8 mit davon gelöstem Halter,
- Fig. 17: eine Darstellung des Einführens der Bandscheibenprothese gemäß Figur 8 in einen Zwischenwirbelraum,
- Fig. 18: eine weitere Darstellung des Einsetzens der Bandscheibenprothese gemäß Figur 8 in einen Zwischenwirbelraum,
- Fig. 19: eine weitere Darstellung des Einsetzens der Bandscheibenprothese gemäß Figur 8 in einen Zwischenwirbelraum,
- Fig. 20: eine weitere Darstellung des Einsetzens der Bandscheibenprothese gemäß Figur 8 in einen Zwischenwirbelraum,
- Fig. 21: eine weitere Darstellung des Einsetzens der Bandscheibenprothese gemäß Figur 8 in einen Zwischenwirbelraum,
- Fig. 22: die Bandscheibenprothese gemäß Figur 8 im in den Zwischenraum eingesetzten Zustand mit schematischer perspektivischer Darstellung einer Knochenschraube,
- Fig. 23: eine Seitenansicht der Bandscheibenprothese 8 im in den Zwischenraum zwischen zwei benachbarten Wirbeln eingesetzten Zustand mit schematischer Darstellung der Knochenschraube,
- Fig. 24: ein siebtes Ausführungsbeispiel einer Bandscheibenprothese mit Halter,
- Fig. 25: die Bandscheibenprothese gemäß Figur 24 mit Halter,
- Fig. 26: die Bandscheibenprothese gemäß Figur 24 mit einem alternativen Federelement und
- Fig. 27: die Bandscheibenprothese gemäß Figur 26 in einer weiteren Position.

In allen Figuren bezeichnen gleiche Bezugsziffern stets gleiche Teile, wobei zur besseren Übersicht oft nicht sämtliche Bezugsziffern in jeder Figur angegeben sind.

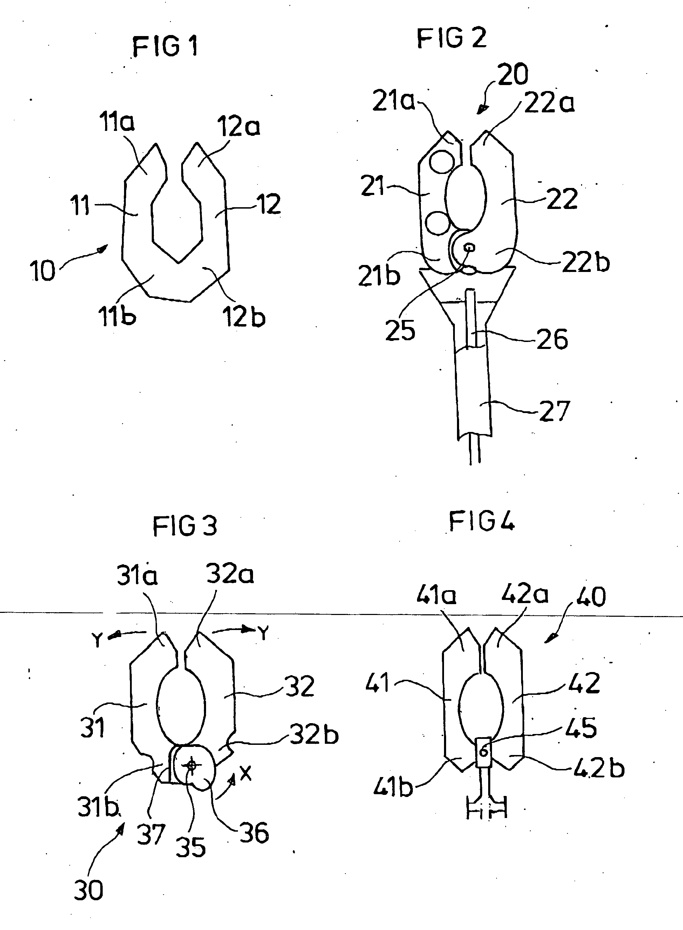

Figur 1 zeigt eine Draufsicht auf ein erstes Ausführungsbeispiel einer Bandscheibenprothese 10 mit einem ersten Schenkel 11, welcher ein erstes Ende 11a und ein zweites Ende 11b aufweist, und einem zweiten Schenkel 12, welcher ein erstes Ende 12a und ein zweites Ende 12b aufweist. Die beiden Schenkel 11, 12 sind an ihrem zweiten Ende 11b, 12b einstückig miteinander verbunden. Die freien Enden 11a, 12a sind aufeinander zugebogen, sodass sich nahezu ein geschlossener Ring mit einem Schlitz ergibt. Die Bandscheibenprothese 10 ist aus einem elastischen Material gefertigt, was es ermöglicht, dass die beiden Schenkel 11, 12 gegeneinander verschwenkt werden können. Die Verschwenkung der beiden Schenkel 11, 12 gegeneinander erfolgt in der Ebene, in der das U-förmige Element liegt. Vorliegend ist dies insbesondere die Papierebene.

Figur 2 zeigt eine Draufsicht auf ein zweites Ausführungsbeispiel einer Bandscheibenprothese 20 mit einem ersten Schenkel 21, welcher ein erstes Ende 21a und ein zweites Ende 21b aufweist, und einem zweiten Schenkel 22, welcher ein erstes Ende 22a und ein zweites Ende 22b aufweist. Die ersten Enden 21a, 22a sind als freie Enden ausgebildet, während die beiden Schenkel 21, 22 an ihren zweiten Enden 21b, 22b miteinander verbunden sind. Diese Verbindung erfolgt mittels eines Bolzens 25. Bei dem Ausführungsbeispiel gemäß Figur 2 sind die beiden Schenkel 21, 22 direkt über einen einzigen Bolzen 25 drehbar gegeneinander gelagert. Alternativ ist es auch möglich, jeden der beiden Schenkel 21, 22 über einen separaten Bolzen an einem Verbindungselement schwenkbar anzuordnen. Der Bolzen 25 verläuft dabei im Wesentlichen senkrecht zur Ebene der im Wesentlichen U-förmigen Bandscheibenprothese 20, in der Darstellung insbesondere senkrecht zur Papierebene. Dadurch werden die beiden Schenkel 21, 22 in der Ebene der U-förmigen Bandscheibenprothese 20, d. h. der Papierebene gegeneinander verschwenkbar gelagert. Ein Aufschwenken der beiden Schenkel 21, 22 gegeneinander kann beispielsweise dadurch erfolgen, dass ein Aufspreizelement 26 eines Halters 27 zwischen die beiden Schenkel 21, 22 geschoben wird und die beiden Schenkel 21, 22 an ihrem jeweiligen zweiten Ende 21b, 22b mit einer derartigen Zwangskurve ausgeformt sind, dass sie dem Aufspreizelement 26 ausweichen und dabei relativ gegeneinander verschwenkt werden.

Figur 3 zeigt eine Draufsicht auf ein drittes Ausführungsbeispiel einer Bandscheibenprothese 30 mit einem ersten Schenkel 31, welcher ein erstes Ende 31a und zweites Ende 31b aufweist, und einem zweiten Schenkel 32, welcher ein erstes Ende 32a und ein zweites Ende 32b aufweist. Die beiden Schenkel 31, 32 sind ähnlich wie in dem zweiten Ausführungsbeispiel mittels eines Bolzens 35 schwenkbar gegeneinander gelagert angeordnet, wobei der Bolzen 35 im Wesentlichen senkrecht zur Ebene der im Wesentlichen U-förmigen Bandscheibenprothese 30 verläuft, d. h. in der vorliegenden Darstellung im Wesentlichen senkrecht zur Papierebene. Ein Aufschwenken der beiden Schenkel 31, 32 gegeneinander erfolgt bei dem dritten Ausführungsbeispiel mittels eines an der Bandscheibenprothese 30 angeordneten Hebelelements 36. Das Hebelelement 36 ist ebenfalls um den Bolzen 35 schwenkbar gelagert angeordnet und beispielsweise als Exenterelement ausgebildet. Bei Schwenken des Hebelelements 36 in Schwenkrichtung X greift das Hebelelement 36 beispielsweise an einer an dem zweiten Ende 31b des ersten Schenkels 31 angeordneten Zwangskurve 37 an, um die beiden Schenkel 31, 32 in Schwenkrichtung Y auseinander zu schwenken. Auch an dem freien Ende 32b des zweiten Schenkels 32 kann eine entsprechende Zwangskurve angeordnet sein, welche ein Auseinanderschwenken der beiden Schenkel 31, 32 bei Schwenken des Hebelelements 36 um den Bolzen 35 bewirkt.

Figur 4 zeigt eine Draufsicht auf ein viertes Ausführungsbeispiel einer Bandscheibenprothese 40 mit einem ersten Schenkel 41, der ein erstes Ende 41a und ein zweites Ende 41b aufweist, und einem zweiten Schenkel 42, der ein erstes Ende 42a und ein zweites Ende 42b aufweist, wobei die beiden Schenkel 41, 42 über ein Zylindergelenk 45 miteinander verbunden sind. In das Zylindergelenk 45 kann ein Betätigungselement 46 eingesetzt werden, um die beiden Schenkel 41, 42 gegeneinander zu verschwenken.

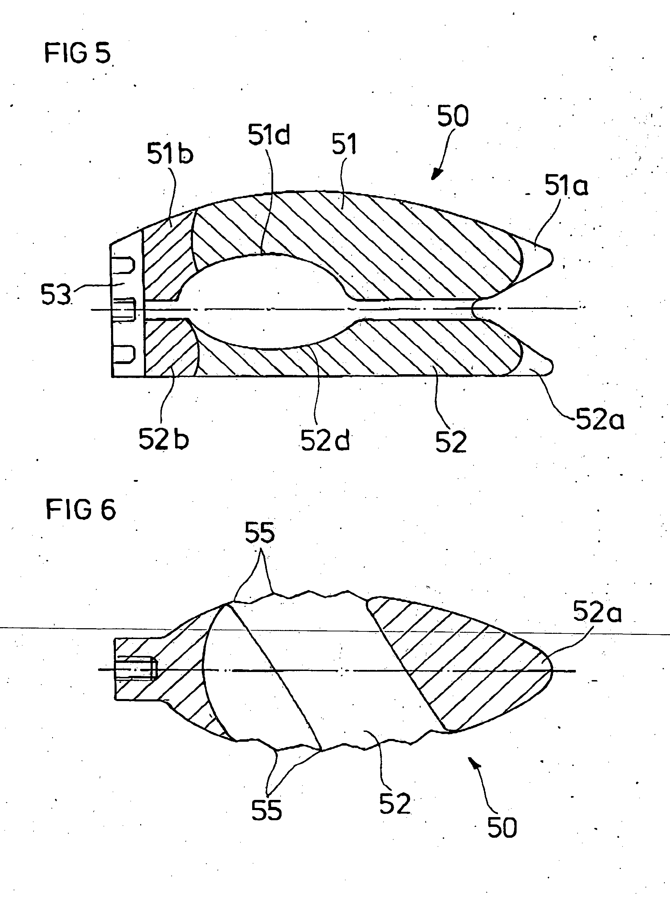

Die Figuren 5 bis 7 zeigen ein erfindungsgemäßes fünftes Ausführungsbeispiel einer Bandscheibenprothese 50. Die Bandscheibenprothese 50 weist einen ersten Schenkel 51 und einen zweiten Schenkel 52 auf, wobei der erste Schenkel 51 ein erstes Ende 51a und ein zweites Ende 51b aufweist, während der zweite Schenkel 52 ein erstes Ende 52a und ein zweites Ende 52b aufweist. Die beiden Schenkel 51, 52 sind an ihrem zweiten Ende 51b, 52b über ein Verbindungselement 53 miteinander verbunden. Dabei ist der erste Schenkel 51 über einen Bolzen 53a an dem Verbindungselement 53 schwenkbar gelagert, während der zweite Schenkel 52 über einen Bolzen 52b an dem Verbindungselement 53 schwenkbar gelagert ist. Weiterhin sind die zweiten Enden 51b, 52b der Schenkel 51, 52 mit einem Zahnradabschnitt 51c, 52c ausgestattet, wobei die Zahnradabschnitte 51c, 52c insbesondere konzentrisch um den jeweiligen Bolzen 53a, 53b angeordnet sind. Beim Einführen der Bandscheibenprothese 50 in einen Zwischenwirbelraum liegen die beiden Schenkel 51, 52 im Wesentlichen parallel (vergleiche Figur 5). Nach Einführen der Bandscheibenprothese 50 in den Zwischenwirbelraum wird ein Aufspreizelement 56 zwischen die beiden zweiten Enden 51b, 52b der Schenkel 51, 52 eingeführt. Das Aufspreizelement 56 weist einen mit den Zahnradabschnitten 51c, 52c der Schenkel 51, 52 korrespondierenden Zahnradabschnitt 57 auf, mittels welchem die beiden Schenkel 51, 52 auseinandergeschwenkt werden, je weiter das Aufspreizelement 56 zwischen die beiden Schenkel 51, 52 eingeführt wird.

Um das Einführen und Positionieren der Bandscheibenprothese 50 in dem Zwischenwirbelraum visuell nachfolgen zu können, sind insbesondere die ersten Enden 51a, 52a mit einem Röntgenkontrastmaterial, beispielsweise mit Tantal beschichtet. Vorzugsweise ist an dritter Stelle ein weiterer Röntgenkontrastmarker angeordnet. Bevorzugt sind wenigstens drei Röntgenkontrastmarker an der Bandscheibenprothese 50 angeordnet, die auch nicht notwendigerweise an den freien Enden 51a, 52a der Schenkel 51, 52 angeordnet sein müssen. Selbstverständlich können die Röntgenkontrastmarker auch bei sämtlichen anderen in der vorliegenden Anmeldung beschriebenen Bandscheibenprothesen verwendet werden.

Die Schenkel 51, 52 weisen an den einander zugewandten Seitenflächen jeweils eine Aussparung 51d, 52d auf. Durch diesen Bereich kann insbesondere eine Knochenschraube, die zwei benachbarte Wirbelkörper gegeneinander stabilisiert, geführt werden.

Wie in der Seitenansicht gemäß Figur 6 erkennbar, können die Ober- und Unterseiten der Schenkel 51, 52 gewölbt ausgebildet sein, um sich an die anatomischen Gegebenheiten anzupassen.

Insbesondere weisen die Ober- und/oder Unterseite der Bandscheibenprothese 50 Zähne 55 auf, um ein Verankern der Bandscheibenprothese 50 in dem benachbarten Wirbelkörpern zu verbessern.

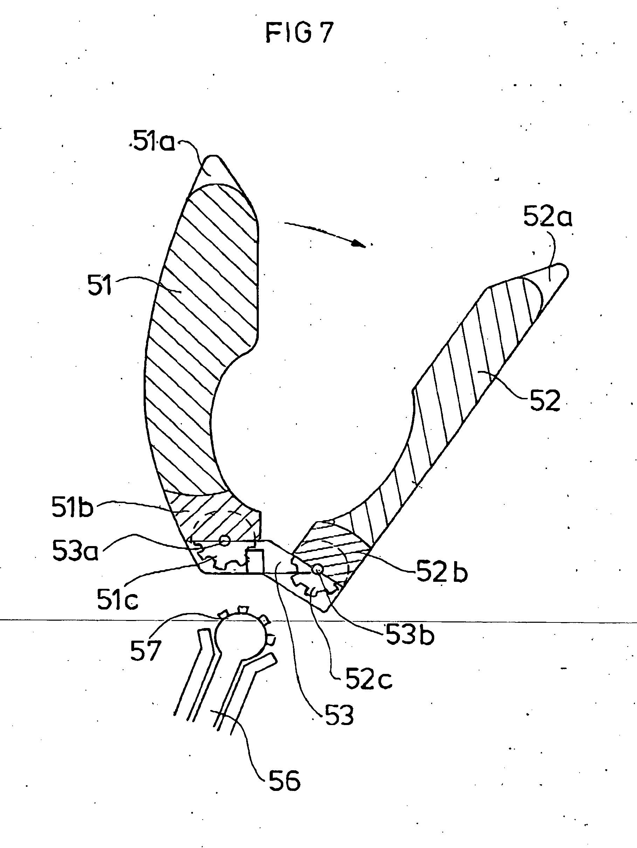

Der erste Schenkel 51 weist vorliegend eine größere Seite auf als der Schenkel 52 (vergleiche Figuren 5 und 7), um für den Fall, dass die Knochenschraube, die die beiden benachbarten Wirbel miteinander verbindet, nicht zentrisch durch den Zwischenwirbelraum geführt ist, eine gleichmäßige Stabilisierung der beiden benachbarten Wirbelkörper gegeneinander zu erreichen.

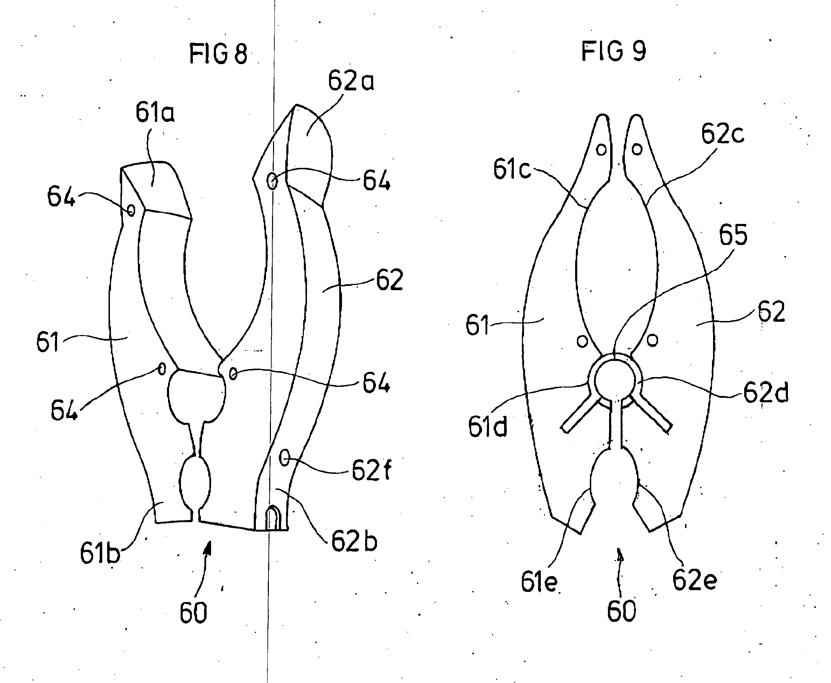

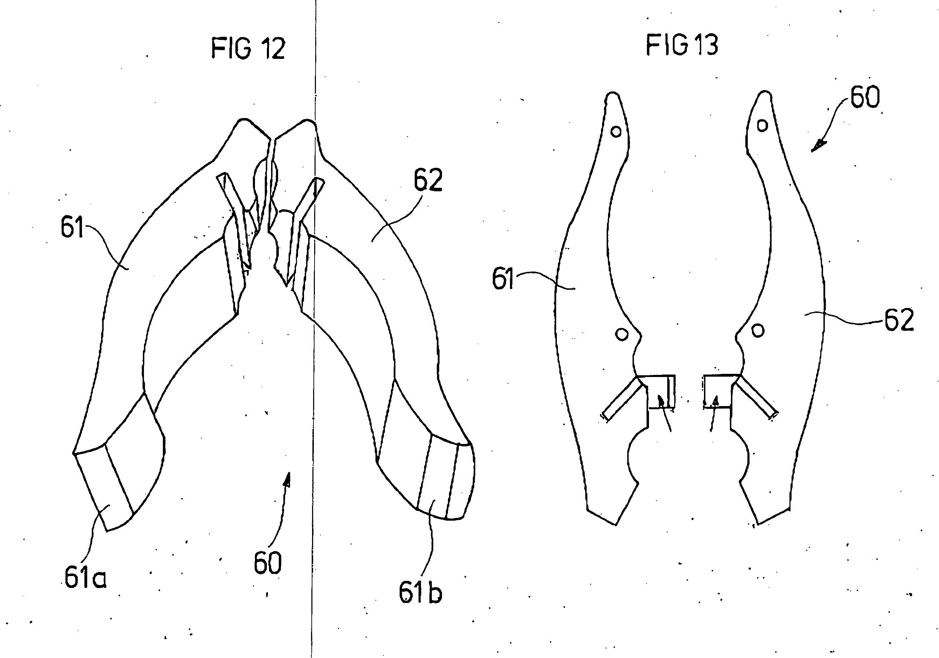

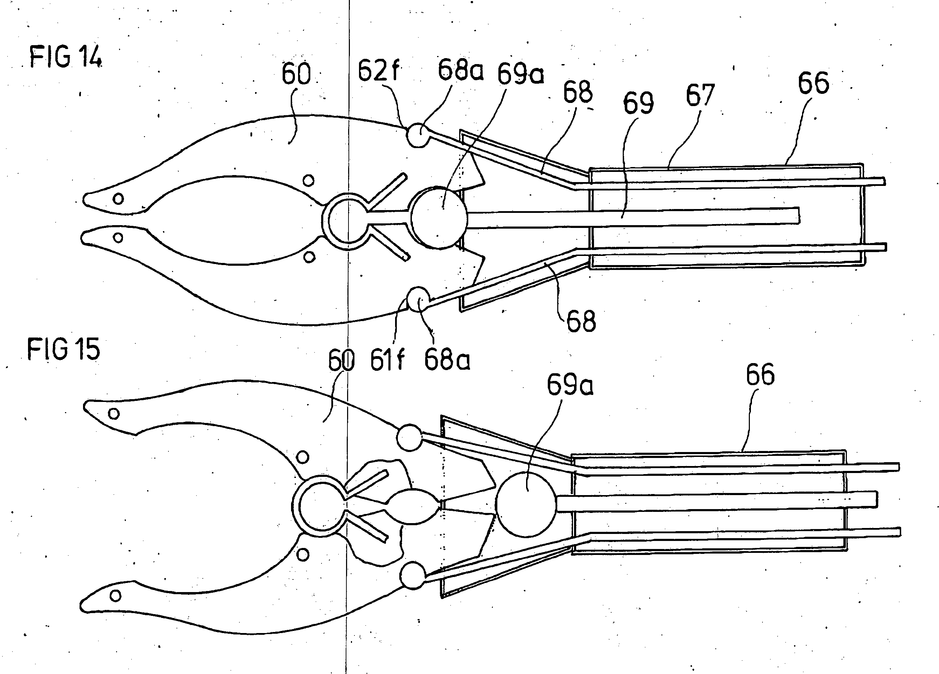

Die Figuren 8 bis 13 zeigen verschiedene Ansichten eines sechsten Ausführungsbeispiels einer Bandscheibenprothese 60, während die Figuren 14 bis 23 darstellen, auf welche Art und Weise die Bandscheibenprothese 60 in den Zwischenwirbelraum eingeführt werden kann.

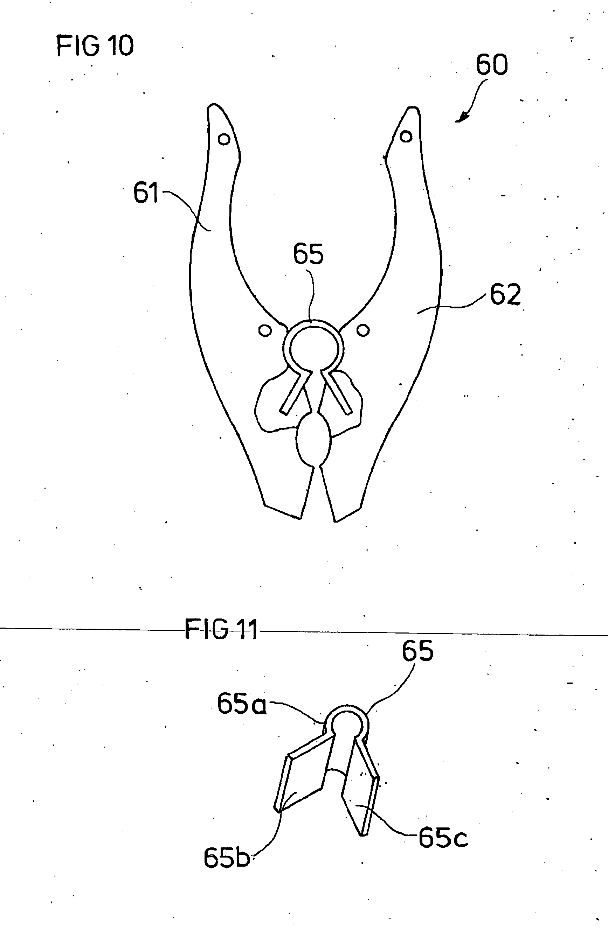

Die Bandscheibenprothese 60 weist einen ersten Schenkel 61 und einen zweiten Schenkel 62 auf, wobei der erste Schenkel 61 ein erstes Ende 61a und ein zweites Ende 61b aufweist, während der zweite Schenkel 62 ein erstes Ende 62a und ein zweites Ende 62b aufweist. Die beiden Schenkel 61, 62 weisen jeweils an der einander zugewandten Seitenfläche jeweils zwischen dem ersten Ende 61a, 62a und dem zweiten Ende 61b, 62b eine zweite Ausnehmung 61d, 62d auf. Die beiden Schenkel 61, 62 sind über ein Federelement 65 (vgl. Fig. 11) miteinander verbunden, welches wie nachfolgend beschrieben, in die zweiten Ausnehmungen 61d, 62d der Schenkel 61, 62 eingreift. Das Federelement 65 weist einen im Wesentlichen zylindrischen Abschnitt 65a auf, welcher über die gesamte Länge geschlitzt ist, und an welchem ausgehend von dem Schlitz zwei Verankerungsflügel 65b, 65c angeordnet sind. Die Verankerungsflügel 65b, 65c sind somit im Wesentlichen um die Längsachse des zylindrischen Abschnitts 65a des Federelements 65 verschwenkbar. Jeweils ein Verankerungsflügel 65b, 65c greift in eine der zweiten Ausnehmung 61d, 62d der Schenkel 61, 62 ein (vergleiche insbesondere Figuren 9 und 10). Dabei zeigt Figur 10 den Zustand des Federelements 65 ohne äußere Krafteinwirkung. Das Federelement 65 ist somit entspannt, wenn die beiden Schenkel 61, 62 gegeneinander aufgespreizt sind. Figur 9 hingegen zeigt das Federelement 65 im belasteten Zustand. Gegen die Kraft des Federelements 65 sind die beiden Schenkel 61, 62 in eine geschlossene Position überführt, in welcher sie insbesondere im Wesentlichen parallel zueinander verlaufen.

Zwischen den zweiten Ausnehmungen 61d, 62d und den ersten Enden 61a, 62a der Schenkel 61, 62 ist auf den einander zugewandten Seitenflächen der Schenkel 61, 62 eine erste Ausnehmung 61c, 62c angeordnet, durch welche insbesondere eine die beiden benachbarten Wirbelkörper verbindende Knochenschraube geführt werden kann.

Zwischen den zweiten Ausnehmungen 61d, 62d und den zweiten Enden 61b, 62b der Schenkel 61, 62 ist auf den einander zugewandten Seitenflächen der Schenkel 61, 62 eine dritte Ausnehmung 61e, 62e angeordnet, in welche wie nachfolgend anhand der Figuren 14 und 15 beschrieben, ein Einsetzinstrument 66 eingreifen kann.

An den Schenkeln 61, 62 sind mehrere Röntgenkontrastmarker 64 angeordnet, insbesondere im Bereich der ersten Enden 61a, 62a der Schenkel 61, 62 und im Bereich zwischen den zweiten Ausnehmungen 61d, 62d und den ersten Ausnehmungen 61c, 62c, um das Einsetzen der Bandscheibenprothese 60 visuell nachverfolgen zu können.

Die Figuren 14 bis 16 zeigen detailliert das Einsetzinstrument 66, welches eine Hülse 67 aufweist, in welcher axial verschiebbar zwei Halteelemente 68 und ein Aufspreizelement 69 angeordnet sind. Die Halteelemente 68 weisen an ihrem distalen Ende Greifelemente 68a auf, welche vorliegend als Kugeln oder Zylinder ausgebildet sind, die in auf den dem jeweilig anderen Schenkel 61, 62 abgewandten äußeren Seitenflächen der Schenkel 61, 62 angeordnete vierte Ausnehmungen 61f, 62f im Wesentlichen formschlüssig eingreifen. Die Greifelemente 68a können dabei in den Ausnehmungen 61f, 62f verrasten oder klemmend gehalten werden oder lediglich im Wesentlichen formschlüssig anliegen. Das Aufspreizelement 69 weist an seinem distalen Ende ein Element 69a auf, welches vorliegend ebenfalls als kugelförmiges oder zylindrisches Element 69a ausgebildet sein kann und in die dritte Ausnehmung 61e, 62e der Bandscheibenprothese 60 eingreift.

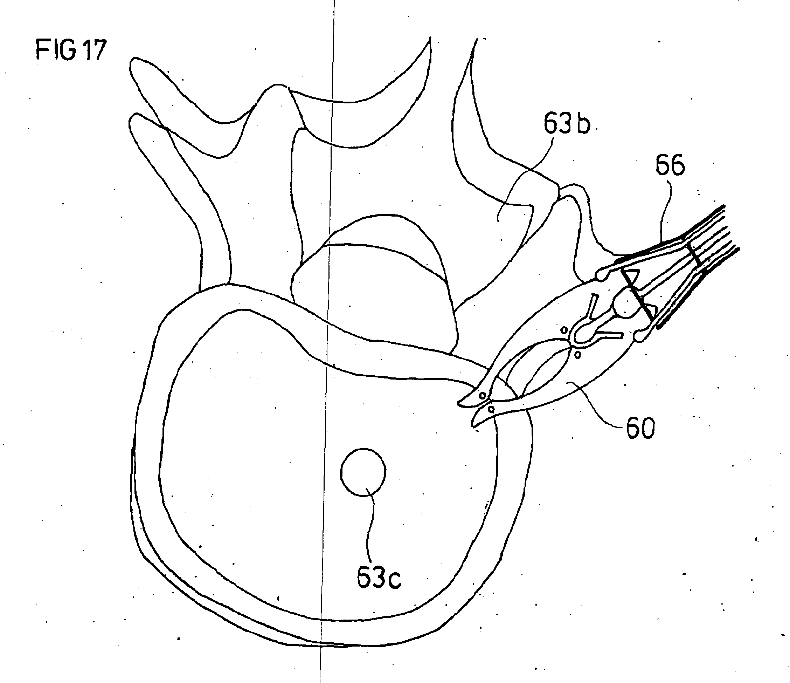

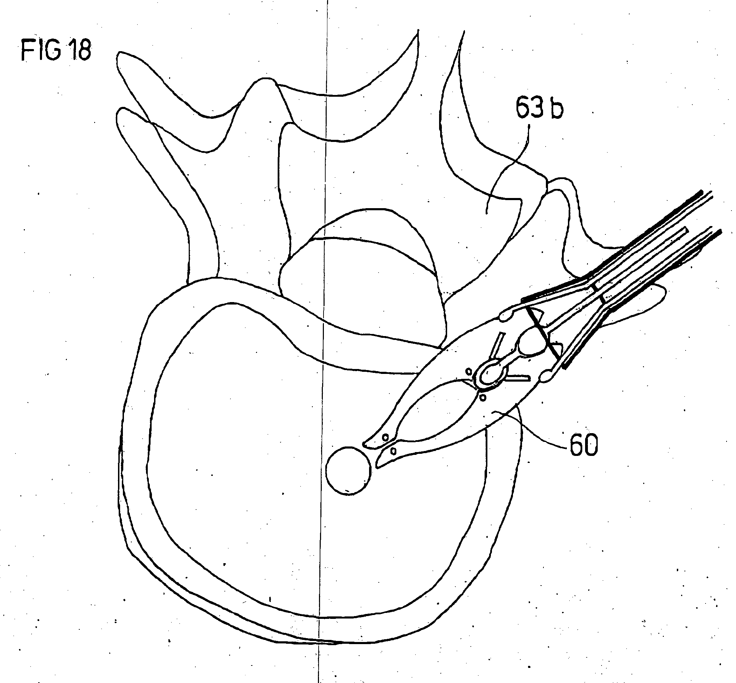

Wie in Figur 14 dargestellt, wird durch Einsetzen des Elements 69a des Aufspreizelements 69 in die dritten Ausnehmungen 61e, 62e das Federelement 65 gegen die Federkraft aufgeweitet und die beiden Schenkel 61, 62 in eine geschlossene Position überführt. Gleichzeitig greifen die Greifelemente 68a der Halteelemente 68 in die vierten Ausnehmungen 61f, 62f der Schenkel 61, 62 ein, um die Bandscheibenprothese 60 zu halten. In dieser Position kann die Bandscheibenprothese 60, wie in den Figuren 17 und 18 gezeigt, in den Zwischenwirbelraum zwischen zwei benachbarten Wirbelkörpern 63a, 63b eingeführt werden. Um die Bandscheibenprothese aufzuspreizen, wird das Aufspreizelement 69 axial in der Hülse 67 des Einsetzinstruments 66 zurückgezogen, sodass durch die Kraft des Federelements 65 die

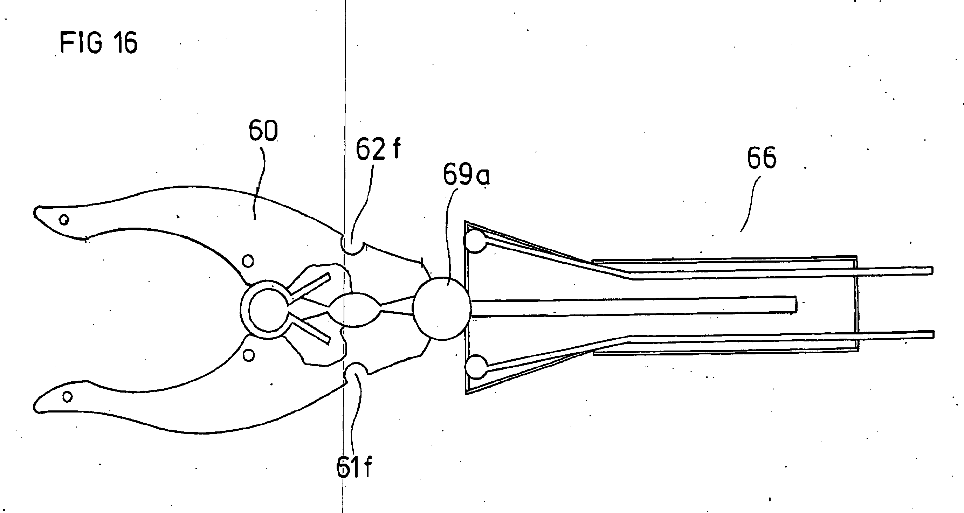

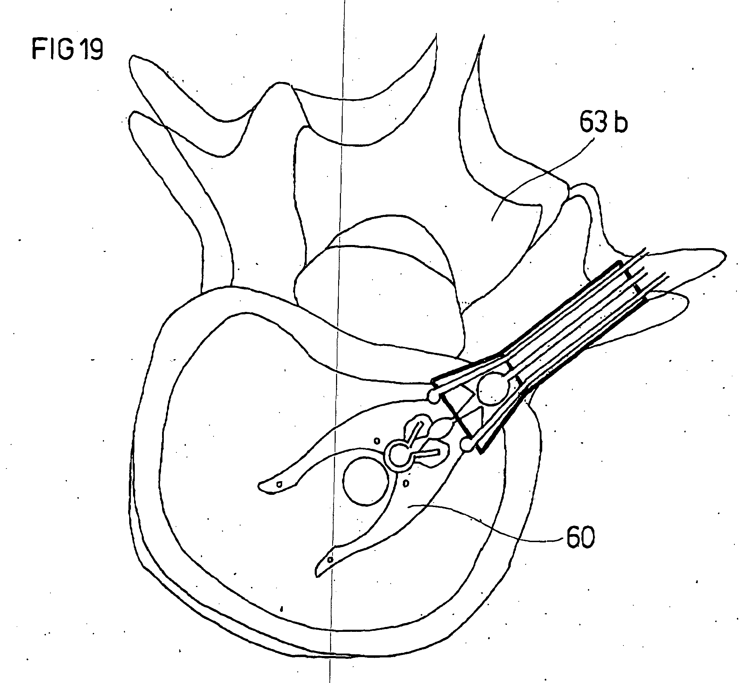

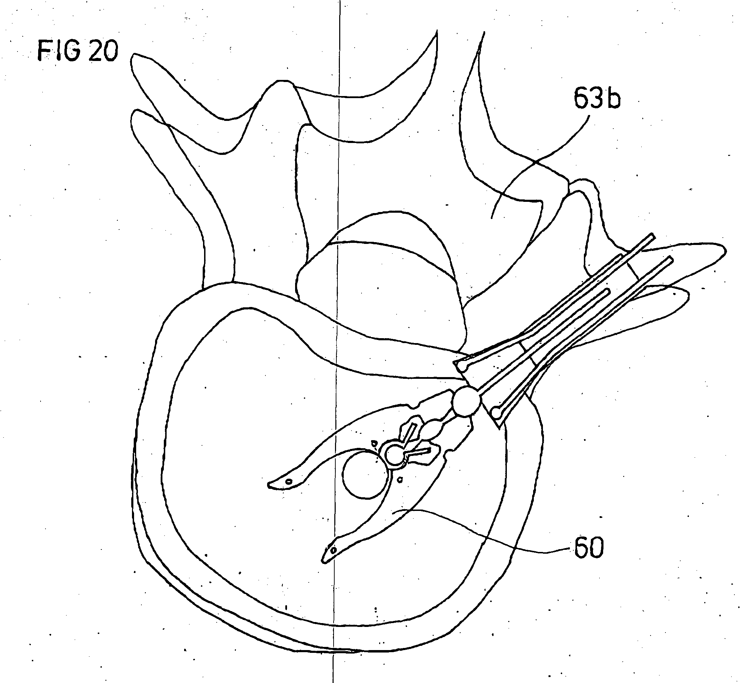

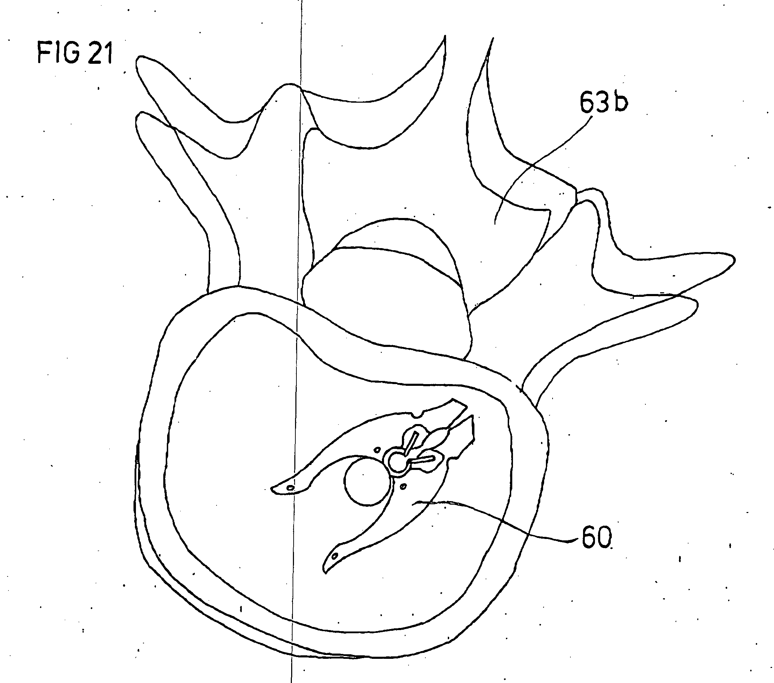

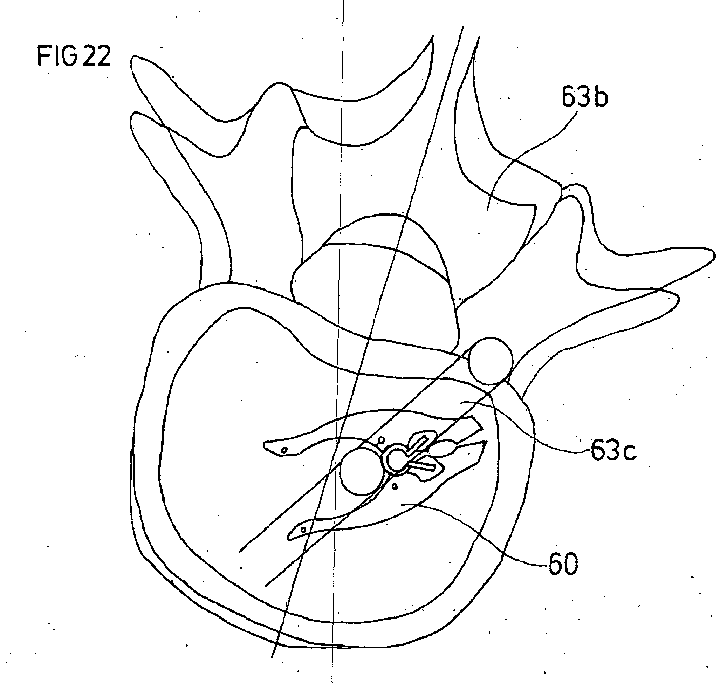

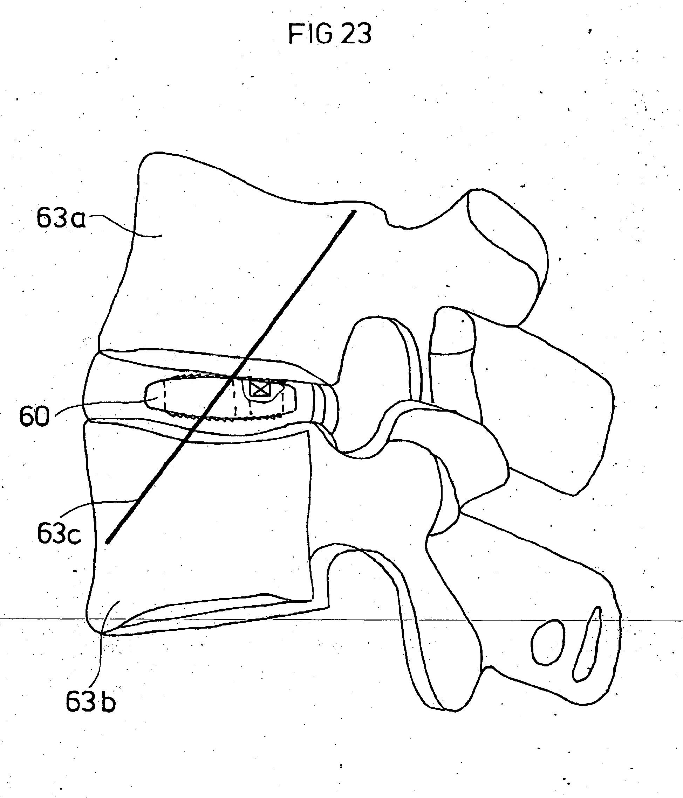

Schenkel 61, 62 gegeneinander aufgespreizt werden (vergleiche Figur 15 und Figur 19). Mittels der Halteelemente 68 kann die Bandscheibenprothese 60 nachfolgend an die gewünschte Position in den Zwischenwirbelraum verschoben werden (vergleiche Figur 19). Anschließend können auch die Halteelemente 68 durch axiales Herausziehen der Halteelemente 68 in der Hülse 67 des Einsetzinstruments 66 von der Bandscheibenprothese 60 gelöst werden (vergleiche Figuren 16 und 20) und nachfolgend das Einsetzinstrument 66 vollständig aus dem Operationsbereich entfern werden (vergleiche Figur 21). Figur 22 verdeutlicht, auf welche Art und Weise eine Knochenschraube 63c den Zwischenwirbelraum und die Bandscheibenprothese 60 insbesondere im Bereich der ersten Ausnehmungen 61c, 62c der Schenkel 61, 62 quer durchsetzt, während in den Figuren 17 bis 21 die Knochenschraube 63c lediglich im Schnitt dargestellt wird. Eine Seitenansicht der Einbausituation der Bandscheibenprothese 60 in den Zwischenwirbelraum zwischen den beiden benachbarten Wirbelkörpern 63a, 63b zeigt Figur 23.

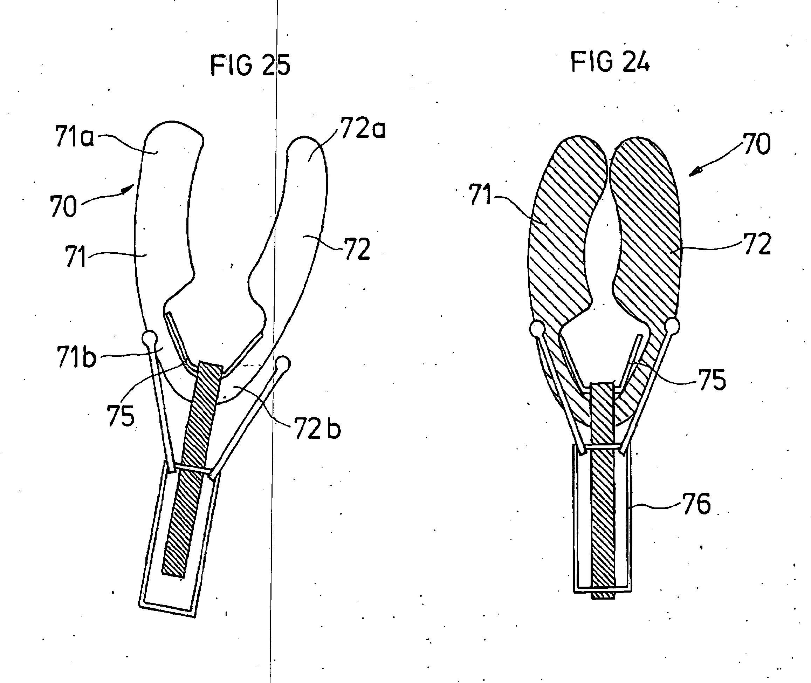

In den Figuren 24 bis 27 ist ein siebtes Ausführungsbeispiel einer Bandscheibenprothese 70 dargestellt, welche einen ersten Schenkel 71 mit einem ersten Ende 71a und einem zweiten Ende 71b sowie einen zweiten Schenkel 72 mit einem ersten Ende 72a und einem zweiten Ende 72b aufweist. Die beiden Schenkel 71, 72 können einstückig miteinander verbunden oder als separater Schenkel ausgebildet sein. Statt des Federelements 65 gemäß dem sechsten Ausführungsbeispiel ist zwischen den beiden Schenkeln 71, 72 ein Federelement 75 aus einer Memorylegierung angeordnet. Die Memorylegierung ist insbesondere derart ausgestalte, dass die Form bei Erreichen der Körpertemperatur geändert wird. Figur 24 zeigt eine Bandscheibenprothese 70 mit den beiden Schenkeln 71, 72 in der geschlossenen Position, in welcher die Bandscheibenprothese 70 in den Zwischenwirbelraum eingeführt werden kann. Dies erfolgt insbesondere bei einer Temperatur, die leicht unterhalb der Körpertemperatur liegt, insbesondere bei Raumtemperatur, sodass das Federelement 75 an den beiden Schenkeln 71, 72 anliegt und gegebenenfalls zusätzlich das Halten der Schenkel 71, 72 in der geschlossenen Position unterstützt. Figur 25 zeigt das Aufweiten des Federelements 75 bei Erreichen der Körpertemperatur auf Grund des Memoryeffekts, wobei das Federelement 75 sich derart aufspreizt, dass die Schenkel, 71, 72 gegeneinander verschwenkt werden.

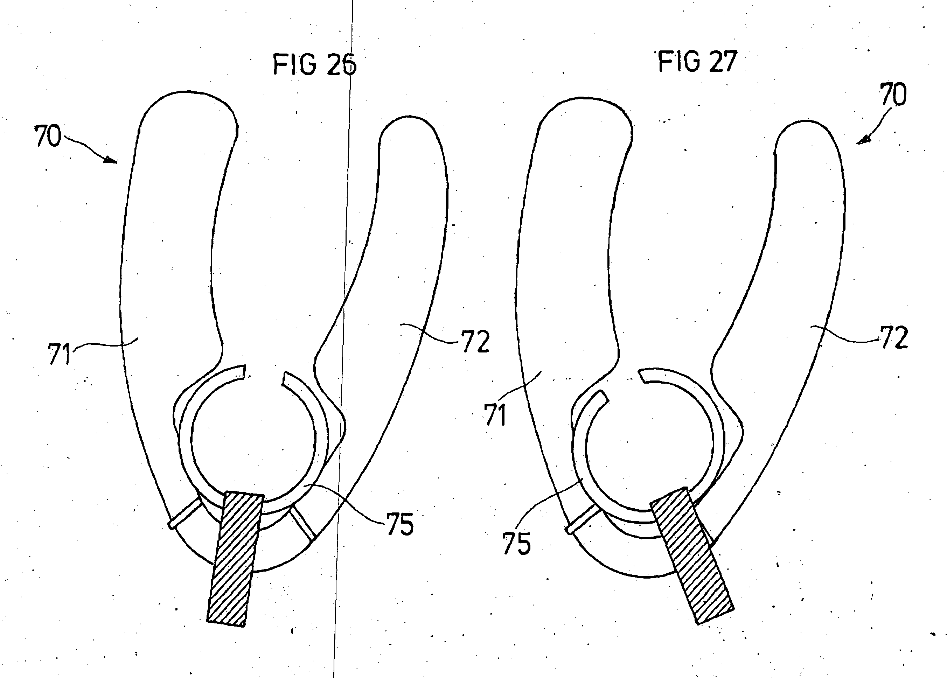

Die Bandscheibe 70 kann anschließend mit einem Einsetzinstrument 76, welches vergleichbar zu dem Einsetzinstrument 66 gemäß dem sechsten Ausführungsbeispiel ist, in die gewünschte Position in dem Zwischenwirbelraum bewegt werden, um das Einsetzinstrument 76 anschließend zu entfernen, wobei die Bandscheibenprothese 70 durch das Federelement 75 in der aufgespreizten Position verbleibt. Das Federelement 75 gemäß der Figuren 24 und 25 weist zwei blattfederschenkelartige Elemente auf. Alternativ kann, wie in den Figuren 26 und 27 dargestellt, das Federelement 75 auch als Schlitzhülse mit zwei gekrümmten Schenkeln ausgebildet sein.

Die Merkmale aus den unterschiedlichen Ausführungsbeispielen können auch beliebig kombiniert werden.

Die Schenkel 11, 12, 21, 22, 31, 32, 41, 42, 51, 52, 61, 62, 71, 72 der Bandscheibenprothesen 10, 20, 30, 40, 50, 60, 70 können sämtlich aus einem elastischen Material gefertigt sein. Besonders bevorzugt sind die Schenkel 11, 12, 21, 22, 31, 32, 41, 42, 51, 52, 61, 62, 71, 72 jedoch aus PEEK (Polyetheretherketon) gefertigt.

### Bezugszeichenliste

- 10: Bandscheibenprothese
- 11: erster Schenkel
- 11a: erstes Ende
- 11b: zweites Ende
- 12: zweiter Schenkel
- 12a: erstes Ende
- 12b: erstes Ende

- 20: Bandscheibenprothese
- 21: erster Schenkel
- 21a: erstes Ende
- 21b: zweites Ende
- 22: zweiter Schenkel
- 22a: erstes Ende
- 22b: zweites Ende
- 25: Bolzen
- 26: Aufspreizelement
- 27: Halter

- 30: Bandscheibenprothese
- 31: erster Schenkel
- 31a: erstes Ende
- 31b: zweites Ende
- 32: zweiter Schenkel
- 32a: erstes Ende
- 32b: zweites Ende
- 35: Bolzen
- 36: Hebelelement
- 37: Zwangskurve
- x: Schwenkrichtung
- y: Schwenkrichtung
- 40: Bandscheibenprothese
- 41: erster Schenkel
- 41a: erstes Ende
- 41b: zweites Ende
- 42: zweiter Schenkel
- 42a: erstes Ende
- 42b: zweites Ende
- 45: Zylindergelenk
- 46: Betätigungselement

- 50: Bandscheibenprothese
- 51: erster Schenkel
- 51a: erstes Ende
- 51b: zweites Ende
- 51c: Zahnradabschnitt
- 51d: Ausnehmung
- 52: zweiter Schenkel
- 52a: erstes Ende
- 52b: zweites Ende
- 52c: Zahnradabschnitt
- 52d: Ausnehmung
- 53: Verbindungselement
- 53a: Bolzen
- 53b: Bolzen
- 55: Zahn
- 56: Aufspreizelement
- 57: Zahnradabschnitt

- 60: Bandscheibenprothese
- 61: Schenkel
- 61a: erstes Ende
- 61b: zweites Ende
- 61c: erste Ausnehmung
- 61d: zweite Ausnehmung
- 61e: dritte Ausnehmung
- 61f: vierte Ausnehmung
- 62: zweiter Schenkel
- 62a: erstes Ende
- 62b: zweites Ende
- 62c: erste Ausnehmung
- 62d: zweite Ausnehmung
- 62e: dritte Ausnehmung
- 62f: vierte Ausnehmung
- 63a: Wirbelkörper
- 63b: Wirbelkörper
- 63c: Knochenschraube
- 64: Röntgenkontrastmarker
- 65: Federelement
- 65a: zylindrischer Abschnitt
- 65b: Verankerungsflügel
- 65c: Verankerungsflügel
- 66: Einsetzinstrument
- 67: Hülse
- 68: Halteelement
- 68a: Greifelement
- 69: Aufspreizelement
- 69a: Element

- 70: Bandscheibenprothese
- 71: Schenkel
- 71a: erstes Ende
- 71b: zweites Ende
- 72: zweiter Schenkel
- 72a: erstes Ende
- 72b: zweites Ende
- 75: Federelement
- 76: Einsetzinstrument

## Patentansprüche

1. Bandscheibenprothese (50) zum Einsetzen zwischen zwei benachbarten Wirbelkörpern (63a, 63b), wobei die Bandscheibenprothese (50) (J-förmig ausgebildet ist mit einem ersten Schenkel (51) und einem zweiten Schenkel (52), wobei die beiden Schenkel (51, 52) relativ zueinander verschwenkbar sind,
**dadurch gekennzeichnet, dass** die beiden Schenkel (51, 52) ein erstes Ende (51a, 52a) und ein zweites Ende (51b, 52b) aufweisen, wobei die beiden Schenkel (51, 52) an ihrem zweiten Ende (51b, 52b) über ein Verbindungselement (53) miteinander verbunden sind, wobei der erste Schenkel (51) über einen Bolzen (53a) an dem Verbindungselement (53) schwenkbar gelagert ist, während der zweite Schenkel (52) über einen Bolzen (53b) an dem Verbindungselement (53) schwenkbar gelagert ist, wobei die zweiten Enden (51b, 52b) der Schenkel (51, 52) mit einem Zahnradabschnitt (51c, 52c)ausgestattet sind und wobei die Zahnradabschnitte (51c, 52c) konzentrisch um den jeweiligen Bolzen (53a, 53b) angeordnet sind, und wobei ein Aufspreizelement (56) mittels eines Zahnradabschnitts (57) zum Schwenken in die Zahnradabschnitte (51c, 52c) eingreifen kann.

2. Bandscheibenprothese nach Anspruch 1,
**dadurch gekennzeichnet, dass** die Bandscheibenprothese (50) aus einem elastischen Material gefertigt ist.

3. Bandscheibenprothese nach einem der vorhergehenden Ansprüche,
**dadurch gekennzeichnet, dass** die beiden Schenkel (52, 51) jeweils über ein Scharnier schwenkbar angeordnet sind.

4. Bandscheibenprothese nach einem der vorhergehenden Ansprüche,
**dadurch gekennzeichnet, dass** die beiden Schenkel (51, 52) jeweils mittels eines Bolzens (53a, 53b) schwenkbar gelagert angeordnet sind.

5. Bandscheibenprothese nach einem der vorhergehenden Ansprüche,
**dadurch gekennzeichnet, dass** die beiden Schenkel mittels eines Federelements , insbesondere eines Federelements aus einer Memorylegierung, verschwenkbar sind.

6. Bandscheibenprothese nach einem der vorhergehenden Ansprüche,
**dadurch gekennzeichnet, dass** an dem einen der beiden Schenkel ein Hebelelement schwenkbar gelagert angeordnet ist, welches bei Drehung um eine Drehachse an einer an dem anderen der beiden Schenkel angeordneten Zwangskurve angreift, um die beiden Schenkel relativ zueinander zu verschwenken.

7. Bandscheibenprothese nach einem der vorhergehenden Ansprüche,
**dadurch gekennzeichnet, dass** die freien Enden (51a, 52a) der Schenkel (51, 52) aufeinander zu gebogen sind.

8. Bandscheibenprothese nach einem der vorhergehenden Ansprüche,
**dadurch gekennzeichnet, dass** wenigstens zwei, vorzugsweise drei, Röntgenkontrastmarker (64) an der Bandscheibenprothese (50) angeordnet sind.

9. Bandscheibenprothese nach einem der vorhergehenden Ansprüche,
**dadurch gekennzeichnet, dass** die beiden Schenkel (51, 52) jeweils ein freies Ende (51a, 52a) aufweisen, welches mit einem Röntgenkontrastmaterial, beispielsweise mit Tantal, beschichtet ist.

10. Bandscheibenprothese nach einem der vorhergehenden Ansprüche,
**dadurch gekennzeichnet, dass** einer der beiden Schenkel (51) in der Ebene der U-förmigen Bandscheibenprothese (50) eine größere Breite aufweist als der andere der beiden Schenkel (52).

11. Bandscheibenprothese nach einem der vorhergehenden Ansprüche,
**dadurch gekennzeichnet, dass** die Bandscheibenprothese (50) wenigstens eine Ausnehmung aufweist, in welche ein Einsetzinstrument, (66, ) eingreifen kann.

## Claims

1. Spinal disc prosthesis (50) for inserting between two adjacent vertebral bodies (63a, 63b), said spinal disc prosthesis (50) being designed in U-shape with a first leg (51) and a second leg (52), wherein the two legs (51, 52) can be pivoted relative to each other, **characterized in that** the two legs (51, 52) have a first end (51a, 52a) and a second end (51b, 52b), the two legs (51, 52) at their second end (51b, 52b) being connected by a connecting element (53), wherein the first leg (51) is pivotably mounted on the connecting element (53) by a bolt (53a), while the second leg (52) is pivotably mounted on the connecting element (53) by a bolt (53b), the second ends (51b, 52b) of the legs (51, 52) being equipped with a toothed wheel section (51c, 52c), wherein the toothed wheel sections (51c, 52c) are concentrically arranged about the respective bolt (53a, 53b), and wherein a spreading element (56) can engage into the toothed wheel sections (51c, 52c) by a toothed wheel portion (57) in a pivotable manner.

2. Spinal disc prosthesis according to claim 1, **characterized in that** the spinal disc prosthesis (50) is made from elastic material.

3. Spinal disc prosthesis according to any one of the above claims, **characterized in that** the two legs (52, 51) are each pivotably mounted by a hinge.

4. Spinal disc prosthesis according to any one of the above claims, **characterized in that** the two legs (51, 52) are each pivotably mounted by a bolt (53a, 53b).

5. Spinal disc prosthesis according to any one of the above claims, **characterized in that** the two legs can be pivoted by a spring element, particularly a spring element made of memory alloy.

6. Spinal disc prosthesis according to any one of the above claims, **characterized in that** a lever element is mounted at one of the two legs in a pivotable manner, said lever element when rotated about a rotational axis acts on a constraint curve mounted at the other of the two legs, so that the two legs pivot relative to each other.

7. Spinal disc prosthesis according to any one the above claims, **characterized in that** the free ends (51a, 52a) of the legs (51, 52) are bent towards each other.

8. Spinal disc prosthesis according to any one of the above claims, **characterized in that** at least two, preferably three, X-ray contrast markers (64) are arranged on the spinal disc prosthesis (50).

9. Spinal disc prosthesis according to any one of the above claims, **characterized in that** the two legs (51, 52) each have a free end (51a, 52a), which is coated with an X-ray contrast material, for example tantalum.

10. Spinal disc prosthesis according to any one of the above claims, **characterized in that** one of the two legs (51) in the plane of the U-shaped spinal disc prosthesis (50) has a greater width than the other of the two legs (52).

11. Spinal disc prosthesis according to any one of the above claims, **characterized in that** the spinal disc prosthesis (50) has at least one recess, into which an insertion instrument (66) can engage.

## Revendications

1. Prothèse de disque intervertébral (50) destinée à être insérée entre deux corps vertébraux voisins (63a, 63b), cette prothèse (50) étant en forme de U et comportant une première branche (51) et une seconde branche (52), ces deux branches (51, 52) pouvant pivoter l'une par rapport à l'autre, **caractérisée en ce que** les deux branches (51, 52) comportent une première extrémité (51a, 52a) et une seconde extrémité (51b, 52b), les deux branches (51, 52) étant reliées entre elles au niveau de leur seconde extrémité (51b, 52b) au moyen d'un élément de liaison (53), la première branche (51) étant montée pivotante sur l'élément de liaison (53) par l'intermédiaire d'un tourillon (53a) tandis que la seconde branche (52) est montée pivotante sur l'élément de liaison (53) par l'intermédiaire d'un tourillon (53b), les secondes extrémités (51b, 52b) des branches (51, 52) étant équipées d'un segment de denture (51c, 52c), les segments de denture (51c, 52c) étant positionnés concentriquement autour du tourillon (53a, 53b) respectif et, un élément d'écartement (56) pouvant venir en prise dans les segments de denture (51c, 52c) au moyen d'un segment de denture (57) pour permettre le pivotement.

2. Prothèse de disque intervertébral conforme à la revendication 1,
**caractérisé en ce qu'**
elle est réalisée en un matériau élastique.

3. Prothèse de disque intervertébral conforme à l'une des revendications précédentes,
**caractérisée en ce que**
les deux branches (51, 52) sont respectivement montées pivotantes par l'intermédiaire d'une charnière.

4. Prothèse de disque intervertébral conforme à l'une des revendications précédentes,
**caractérisée en ce que**
les deux branches (51, 52) sont respectivement montées pivotantes au moyen d'un tourillon (53a, 53b).

5. Prothèse de disque intervertébral conforme à l'une des revendications précédentes,
**caractérisée en ce que**
les deux branches peuvent pivoter au moyen d'un élément élastique, en particulier d'un élément élastique en un alliage à mémoire de forme.

6. Disque intervertébral conforme à l'une des revendications précédentes,
**caractérisé en ce que**
sur l'une des deux branches est monté pivotant un élément de levier qui vient en prise lors d'une rotation autour d'un axe de rotation avec une courbe de contrainte située sur l'autre branche pour permettre de faire pivoter les deux branches l'une par rapport à l'autre.

7. Prothèse de disque intervertébral conforme à l'une des revendications précédentes,
**caractérisé en ce que**
les extrémités libres des branches (51, 52) sont destinées à être recourbées l'une sur l'autre.

8. Prothèse de disque intervertébral conforme à l'une des revendications précédentes,
**caractérisé en ce qu'**
elle est équipée d'au moins deux et de préférence de trois repères par contraste aux rayons X (64).

9. Prothèse de disque intervertébral conforme à l'une des revendications précédentes,
**caractérisé en ce que**
les deux branches (51, 52) comprennent chacune une extrémité libre (51a, 52a) qui est recouverte d'un matériau de contraste aux rayons X, par exemple de tantale.

10. Prothèse de disque intervertébral conforme à l'une des revendications précédentes,
**caractérisé en ce qu'**
une des deux branches (51) a dans le plan de la prothèse de disque intervertébral (50) en forme de U une largeur supérieure à celle de l'autre branche (52).

11. Prothèse de disque intervertébral conforme à l'une des revendications précédentes,
**caractérisé en ce qu'**
elle comporte au moins un évidement dans lequel peut venir en prise un instrument d'implantation.
